# EUROPEAN PATENT SPECIFICATION

(11) **EP 3 772 317 B1**
(45) Date of publication and mention of the grant of the patent: **12.07.2023**
(21) Application number: 19190493.7
(22) Date of filing: 07.08.2019
(51) Int. Cl.: A61B 1/05, A61B 1/00, A61B 1/31, A61B 1/12, A61B 1/06, A61B 1/018, A61B 1/015

(54) **ENDOSCOPE**
ENDOSKOP
ENDOSCOPE

(43) Date of publication of application: 10.02.2021
(73) Proprietor: ETH Zürich, 8092 Zürich (CH)
(72) Inventor: Nelson, Bradley J., 8126 ZUMIKON (CH); Lyttle, Sean L., 8610 USTER (CH)
(74) Representative: adares Patent- und Rechtsanwälte Reininger & Partner GmbB

(56) References cited:
- WO-A1-2017/100751
- JP-A- 2006 187 583
- JP-B2- 4 885 634
- US-A1- 2008 033 245

## Description

The invention relates to an endoscope for inspection and study of the gastrointestinal tract, especially the human small intestine.

There is a growing body of evidence suggesting a link between intestinal microbiota and disease. However, current research is severely limited because there is no good way to sample the whole length of the small intestine. The large intestine is relatively easy to access and inspect with modern endoscopic devices, but the inspection of the small intestine, also called the small bowel, poses major anatomical challenges that have yet to be overcome. It is currently very difficult for Gastroenterologists to examine or treat the entirety of the small intestine. While the first 1-2 meters are usually accessible using classical push-endoscopy techniques, the remaining 3-4 meters are notoriously difficult to access non-invasively. Several technologies, such as double balloon endoscopy (DBE) and spiral endoscopy (SE), have tried to address this issue with limited success.. Although these techniques offer some benefits over traditional push endoscopy, they are complex, time-consuming and are still unable to reliably access the entire small intestine.

JP 2006 187583 discloses an endoscope having an outer cylinder and an inner cylinder arranged coaxially inside the outer cylinder. The inner cylinder comprises a thread portion on its outer surface that engages with a thread on the inside surface of the outer cylinder. A distal end of the inner cylinder extends outside of a distal end of the outer cylinder. Both distal ends are covered by a flexible cover made up of two umbrella-like structures. By rotating the inner cylinder against the outer cylinder, the distal ends move against each other either further away or closer together. When the distal end of the inner cylinder moves away from the outer cylinder, the umbrella-like structure is in a folded configuration and allows a forward movement of the distal end. When the distal end of the inner cylinder moves retracts towards the outer cylinder, a hinge-system forces the umbrella-like structure to expand radially and engage with the intestinal wall, forcing the outer cylinder to move towards the distal end of the inner cylinder.

WO 2017/100751 discloses an endoscope having a distal end that is covered by an endoscopic hood made of a translucent or transparent material. The hood has an inner thread that engages with an outer thread of the distal end and allow for the hood to move between an extended and a retracted configuration by rotating the hood against the endoscope. By moving the endoscopic hood from the retracted to the extended configuration, a longitudinal force is to be exerted against folds of the intestinal wall, thereby stretching and exposing target tissue hidden or obscured within the fold.

It is an object of the present invention to provide an endoscope for the inspection and study of the small intestine, which can be used to transverse the greater part or even the entire small intestine in a safe, reliable and reproducible manner.

This objective is met according to the present invention by providing a device with the features of claim 1. Further advantageous embodiments of the invention are subject of the sub-claims.

Unlike traditional endoscopes, the proposed concept does not rely on a proximal pushing force to make progress. Instead, a flexible tubular structure is rotated proximally, and this rotation is gradually propagated through the tubular structure to the distal end of the tubular structure, even through sharp curves and over long distances. An elongated element extends through the distal tip of the tubular structure and connects to an endoscopic tip. The elongated element is rotationally coupled to the tubular structure such that when the tubular structure is rotated in a first direction, the elongated element is translated forward and a gap between the distal end of the tubular structure and the endoscopic tip is increased. The endoscopic tip is thus moved forward further into the small intestine. A rotation of the tubular structure in a second rotational direction translates the elongated element backwards and decreases the gap between the distal end and the endoscopic tip, thereby causing the proximal portions of the tubular structure to travel forward along the elongated element and towards the endoscopic tip. The entire endoscope thus makes forward progress by repeatedly rotating the tubular structure in alternating rotation directions.

With this feature, the proposed endoscope provides means to access the entire length of the small intestine quickly and easily, without undue complexity for the physician and with minimal discomfort for the patient.

To convert rotation of the tubular member into linear motion of the endoscope tip, the endoscope comprises an elongated, flexible tubular structure and a flexible, elongated element disposed inside the tubular structure such that a distal portion of the elongated element protrudes from a distal end of the tubular structure and is connected to the endoscopic tip. The elongated element and the tubular structure may in particular be arranged coaxially or substantially coaxially.

Herein, the expressions proximal and distal are used with respect to the position of the physician utilizing the endoscope. The expression distal thus describes parts, sections or directions that are oriented or placed towards the inside of the patient's body, where the physician cannot manipulate the parts directly with his hands. Likewise, the expression proximal stands for the parts, sections or directions positioned at or oriented towards the location of the physician.

An endoscopic tip is attached to the distal end of the elongated element, and the elongated element is engaged with the tubular structure such that a rotation of the tubular structure around the elongated element in a first rotation direction will increase the gap between the distal end of the tubular structure and the endoscopic tip. Correspondingly, a rotation of the tubular structure around the elongated element in a second rotation direction, opposite to the first rotation direction, will decrease the gap between the endoscopic tip and the tubular structure's distal end. The first rotation direction may be a counterclockwise direction, in which case the second rotation direction is a clockwise direction. Alternatively, the first rotation direction may be a clockwise direction, in which case the second rotation direction is a counterclockwise direction.

The physician utilizing the endoscope will hold a proximal portion of the tubular structure and rotate it alternately in a counterclockwise and a clockwise direction while navigating the small intestine of the patient. Because of the engagement between the elongated element and the tubular structure, this will cause the elongated element to move back and forth along the length of the tubular structure, alternately increasing and decreasing the gap between the endoscopic tip and the distal end of the tubular structure. The endoscopic tip will thus be pushed forward during the first half of the rotation cycle and the tubular structure will be pulled toward the now advanced endoscopic tip during the next half of the rotation cycle.

A gentle bias, i.e. a small pushing force, may be applied to the proximal end of the tubular structure during the rotation cycle. Due to the longitudinal flexibility (compressibility) of the tubular structure, this force causes the proximal portions of the tubular structure to be compressed. This compression propagates through the tubular structure during rotation and encourages the forward motion of the distal end of the tubular structure. Even though regular pushing may not be effective, a rotation of the tubular structure causes a constant stick-slip phenomenon on the intestinal wall, allowing small sections of the tubular structure to make forward progress. Other cumulative or alternative effects for causing or enhancing the forward movement of the endoscope due to this alternating rotation will be explained further below.

According to a preferred embodiment, engagement between the elongated element and the tubular structure is by way of an engaging element engaging with a thread structure or a helical structure. In particular, the engaging element, such as a protruding element like a pin or a peg, may reach into the threads of the thread structure in order to translate a rotation of the tubular member into a translational movement of the elongated element with respect to the engaging element.

The thread structure in a preferred embodiment may be a thread formed on an outside surface of said elongated element. In this case, the engaging element must be attached to the tubular structure. Alternatively, the thread structure may be a thread formed on an inside surface of the tubular structure or otherwise be attached to the tubular structure and the engaging element may be attached to the elongated element, preferably to its outside surface.

In advantageous embodiments, said engagement between the elongated element and the tubular structure may be effected at two or more positions along the length of the elongated element and/or the tubular structure. If the engagement is accomplished with a helical or thread structure, there might therefore be more than one engagement element placed along the threaded structure. Alternatively, the engagement element could also be a second threaded or helical structure interlocking with the threaded or helical structure over a certain length.

In one embodiment, the tubular structure also includes an outer spiraling surface structure. In other words, the outside surface of the tubular structure has a groove or a ridge that spirals along the length of the tubular structure.

The spiraling surface structure may be made of one or several such groove(s) or ridge(s). The spiraling surface structure may be formed over a distal section, over a major part, or over substantially all of the tubular structure. Said spiral surface structure of the tubular structure may act similar to a screw by engaging with the intestinal wall during rotation. Consequently, it can provide a further forward bias during rotation, in order to propel the distal end towards the endoscopic tip during rotation in the second rotation direction.

A further way of facilitating the forward movement of the is to form the endoscopic tip such that it experiences less frictional resistance when being pushed forward than when pulled backward inside the intestine. Thus, the increasing gap between the endoscopic tip and the distal end of the tubular structure will tend to push the endoscopic tip forward within the intestine, while the decreasing gap between the endoscopic tip and the distal end will tend to draw the tubular structure along the intestine forward towards the endoscopic tip, instead of pulling the endoscopic tip back towards the tubular structure.

The endoscopic tip may in a preferred embodiment have substantially the same diameter as the distal end of said tubular structure, at least at the proximal end of the endoscopic tip, i.e. at the end that abuts the distal end when in a completely retracted position.

The endoscope is designed such that when the endoscopic tip is in a completely retracted position, in which the endoscopic tip is abutting the distal end of the tubular structure, a seal is formed between said endoscopic tip and said distal end, sealing an inner lumen of said tubular structure distally, such that substantially no liquid or gas can enter or leave the inner lumen at the distal end.

Besides facilitating the forward movement of the endoscope, the endoscopic tip may contain instruments that aid in the inspection of the small intestine. For example, the endoscopic tip may contain a camera, in particular an endoscopic camera for inspection of the gastro-intestinal tract. Furthermore, lights may be positioned on the endoscopic tip in order to illuminate the intestine for allowing image and video taking with the camera. Another instrument might be an electrode, in particular an electrode disposed on the proximal side of the endoscopic tip. A corresponding counter-electrode may in this case be disposed on the distal-most part of the distal tip of the tubular structure. The electrodes may in particular be used for measuring impedances or potentials. The endoscopic tip may also contain a means for remotely steering the tip, for example a permanent magnet designed to be manipulated with external magnetic fields. The endoscopic tip may also contain a means for sampling the epithelial tissue of the intestinal wall.

Wires for the camera, lights and/or the electrode may run from the endoscopic tip through a central lumen of the elongated element to a proximal end of the endoscope. The second electrode on the distal end, if provided, may be electrically connected to a wire running along or inside a wall of the tubular structure. In particular, it may be part of a reinforcement or spiral reinforcement of the tubular structure wall.

In the embodiment with the first electrode on the proximal side of the endoscopic tip and the second electrode on the distal end of the tubular structure, such that the two electrodes are separated by the gap between the endoscopic tip and the distal end, transepithelial impedance may be measured by advancing the tubular structure until a portion of the intestinal wall is sandwiched between the two electrodes. Then the impedance between the two electrodes is measured, the value of which corresponds to twice the transepithelial impedance. For this impedance measurement, a low pressure or vacuum may be applied to the inner lumen of the tubular structure, causing air to be sucked into the inner lumen from the gap between the endoscopic tip and the tubular structure's distal end, thus pulling the intestinal wall into the gap. For this purpose, the distal end of the inner lumen of the tubular structure is not sealed or not completely sealed from the outside, at least as long as the endoscopic tip is not completely retracted.

In advantageous embodiments, the endoscope includes one or more actuation and/or sensory module(s). The module or modules may be located at one or multiple positions along the length of the tubular structure. If multiple modules are provided, they may be arranged at certain distance intervals along the length of said tubular structure, preferably at regular intervals, in particular along the entire length of the tubular structure. Modules of the same sort, e.g. biopsy sampling modules, may be arranged at regular intervals of between 15 cm and 30 cm, in particular between 20 cm and 25 cm. In this case, there may be between 18 and 36, advantageously between 24 and 30, such modules provided along a 6 m length of the tubular structure. Modules of different sorts may also be arranged in any order along the length of the tubular structure. These modules may be permanently fixed to the tubular structure, or may be modular (i.e. able to be removed and replaced with other modules). In this way, different modules may be arranged in a variety of ways for specific purposes or procedures. Alternatively or in addition, one or more specific modules that are specifically designed to detachably connect separate sections of the endoscope together may be provided.

The module or modules may be actuated mechanically, electrically and/or hydraulically/pneumatically. Two, multiple or all of the modules may be actuated simultaneously by the same triggering event. In particular, all modules of the same sort may be actuated simultaneously.

Preferably, the actuation and/or sensory module(s) is/are from the following group: A biopsy sampling module, an impedance measurement module, a drug or fluid delivery or distribution module, a camera module, a handling module for holding or driving the endoscope, or any combination thereof. The term "any combination thereof" may in this regard mean that a single module has multiple functions integrated into it, such as a drug delivery module which contains a camera for more exactly pinpointing the site of drug delivery. That term may, however, also mean that there are modules with different functions placed at distances along the tubular structure.

The biopsy sampling module is configured to extract a biopsy of epithelial tissue from the intestinal wall. For this purpose, in one embodiment, the sampling module comprises a central shaft, a sampling wheel, a sampling module, a torsion spring, a protective casing, a piston, a piston spring, and an endcap. For purposes of sterility, the sampling module may be disposable. Alternatively, the sampling module may be reusable after a sterilization step. The central shaft may be fixed to the tubular structure and have a lumen through which the elongated element passes. This lumen of the sampling module may be in fluid contact with the inner lumen of the outer tubular structure. The torsion spring is engaged with the central shaft, as well as with the sampling wheel, which is free to rotate around the central shaft. The sampling wheel has a cutout into which a disposable sample holder may be inserted and removed.

The disposable sample holder may consist of a receptacle where the sample is stored, and a blade or brush with which the biopsy is obtained. The casing is fixed to the central shaft and surrounds the sampling wheel, guarding the disposable sample holder when not in use. The casing further has a cutout to allow the sampling blade or brush to contact the tissue when the unit is triggered. The piston is fitted around the central shaft just behind the sampling wheel and is free to move linearly. The piston spring pushes the piston up against the sampling wheel, locking the sampling wheel in place.

When the sample lumen of the sampling module is pressurized, holes in the central shaft allow the air to push the piston back against the piston spring, releasing the sampling wheel and causing the biopsy to be taken. At the end of its stroke, the sample holder has rotated by approximately 180 degrees and is now protected from contamination by the other side of the casing. When the pressure is released, the piston returns to its original position, fixing the sampling wheel in its final position. The endcap is fixed to the central shaft as well as the tubular structure.

The impedance measurement module is configured to measure transepithelial impedance, transepithelial resistance or transepithelial electric potential. For this purpose, it may have one, two or multiple electrodes for contacting the intestinal wall. The drug or fluid delivery or distribution module is configured for localized delivery of a medication or any other substance (e.g. probiotics, microrobots, fluorescent dye, contrast agent etc.) inside the intestine. In addition to or instead of the endoscopic camera placed at the endoscopic tip, one or more camera modules may be provided along the length of the endoscope, facing the intestinal wall. The camera of such a camera module may comprise an image sensor and a clear casing and be configured to take video or still images. As the tubular structure rotates, so will the image sensor, allowing for full 360 imaging of the intestinal wall. The handling module may provide sections that can be engaged with a tool, such as an automatic wrench, which can then aid in rotating and/or pushing the tubular structure, while the handling module is outside of the intestine. For example, the handling module may have one, two or multiple flat sections, on which a wrench or any other suitable tool can be engaged.

According to advantageous embodiments, some of the modules may be pneumatically or hydraulically actuated. In particular, one, multiple or all of said modules may be activated by a change of pressure in an inner lumen of said tubular structure.

In a preferred embodiment, a proximal end of said elongated element extends proximally through a sliding seal and beyond a proximal end of said tubular structure. The sliding seal ensures that the elongated element and tubular structure can move relative to each other without compromising the sealed internal volume of the tubular structure. The elongated element may thus be manipulated manually by the physician from the proximal side of the tubular structure, which is facing the physician. Alternatively, the proximal end of said elongated element may be positioned inside said tubular structure. This means in particular that the elongated element is shorter, potentially much shorter than the tubular structure.

Some examples of embodiments of the present invention will be explained in more detail in the following description with reference to the accompanying schematic drawings, wherein:
- Fig. 1: shows a cut-away view of an endoscope according to a first embodiment;
- Fig. 2: shows a cross-sectional view of an endoscope according to a second embodiment;
- Fig. 3: shows a cross-sectional view of an endoscope according to a third embodiment;
- Fig. 4: shows a cross-sectional view of an endoscope according to a fourth embodiment;
- Fig. 5a-5d: show four different embodiments of actuation and/or sensory modules that can be arranged along the endoscope;
- Fig. 6: shows a cross-sectional view of a biopsy sampling module according to one preferred embodiment; and
- Fig. 7: shows an exploded-view drawing of the biopsy sampling module shown in Fig. 6.

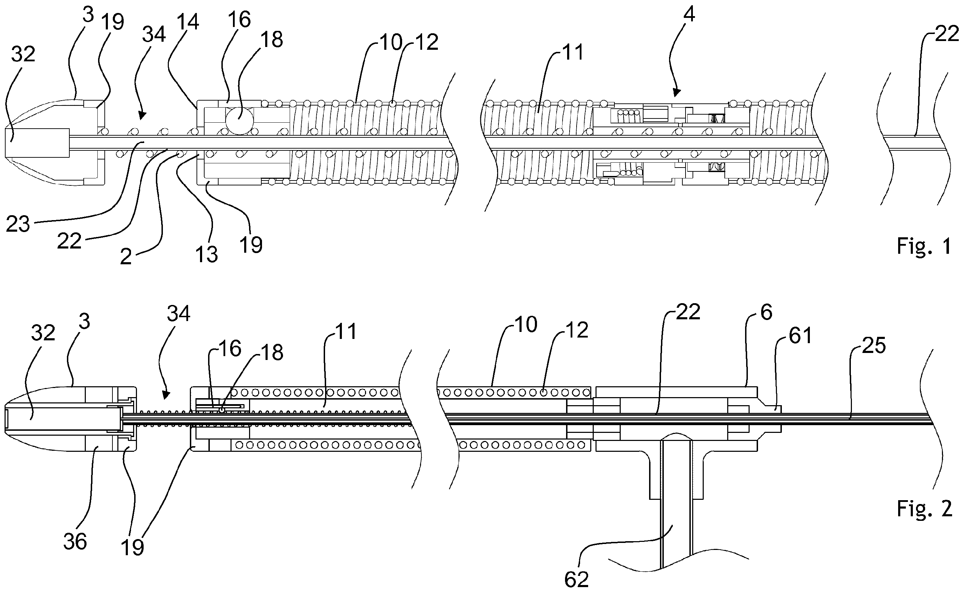

Fig. 1 shows a distal part of an endoscope according to a first embodiment. The endoscope comprises a tubular structure 10, which is made of a metallic spiral reinforcement 12 and a flexible membrane closing the gaps between adjacent sections of the spiral reinforcement 12. The tubular structure 10 surrounds an inner lumen 11, which may be pressurized or depressurized for various functions. The tubular structure 10 ends in a distal end 14, which has a distal opening 13.

An elongated element 22 runs through the inner lumen 11 of the tubular structure 10, and through the distal opening 13, ending in an endoscopic tip 3.

The elongated element 22 is rotationally engaged with the tubular structure 10 such that a relative rotation of the elongated element 22 and the tubular structure 10 will result in a corresponding linear movement between the elongated element 22 and the tubular structure 10 along a joint longitudinal axis.

For providing the rotational engagement, the elongated element 22 is surrounded by a thread structure 2, in particular a spiraling wire, attached to a tube. The tube itself may also be made of a spiraling wire, which is more densely packed than that of the thread structure 2. There is an engaging element in the form of a bearing ball 18 placed inside the distal end 14 of the tubular structure 10. The bearing ball 18 engages with the thread structure 2 to translate rotational movement of the tubular structure around the longitudinal axis into linear or translational movement of the elongated element along the longitudinal axis. The bearing ball 18 is held inside the distal end 14 by a sleeve 16.

The movement of the elongated element 22 with respect to the tubular structure 10 along the longitudinal axis causes the endoscopic tip 3 to move in the same fashion with respect to the tubular structure 10. When the elongated element 22 is retracted as far as possible into the tubular structure 10, the endoscopic tip 3 is in a retracted position (not shown in the figures). In this position, it will cover the opening 13, and, if so configured, also seal the inner lumen 11 at the opening 13, allowing the inner lumen 11 to be pressurized or depressurized. Alternatively, the distal opening 13 may be a sliding seal, which ensures that the inner lumen 11 is sealed against the outside even when the endoscopic tip 3 is not in the retracted position.

When the tubular structure 10 is rotated in one direction (e.g. counterclockwise), the rotational engagement causes the endoscopic tip 3 to move forward, away from the distal end 14. In Fig. 1, the endoscopic tip 3 is shown in an extended position, in which a gap 34 is formed between the endoscopic tip 3 and the distal end 14 of the tubular structure 10. If, at this point, the tubular structure 10 is rotated in the other direction (e.g. clockwise), the rotational engagement causes the tubular structure 10 to move forward along the spiraling wire of the thread structure 2 towards the endoscopic tip 3, thus reducing the gap 34.

The endoscopic tip 3 comprises a camera 32, which is configured to take images or video images of the inside of the intestine. The images may be sent in digital form via wireless connection to an outside device, such as a computer. Alternatively, the camera may be connected via wires, which may run through a central lumen 23 of the elongated element 22. The three embodiments of the endoscope have such wires running through the central lumen 23, as can be seen in Fig. 2-4.

The elongated element 22 of the endoscope shown in Fig. 1 extends beyond the proximal end (not shown in Fig. 1) of the tubular structure 10. As can be seen in Fig. 1 on the right side, the part of the elongated element 22 that extends in such a manner outside of the tubular structure 10, is not necessarily covered by the thread structure 2 anymore. It is only necessary for the thread structure 2 to be disposed on relatively short length of the elongated element's 22 distal end because the "stroke" of the device may be only 20-30 cm.

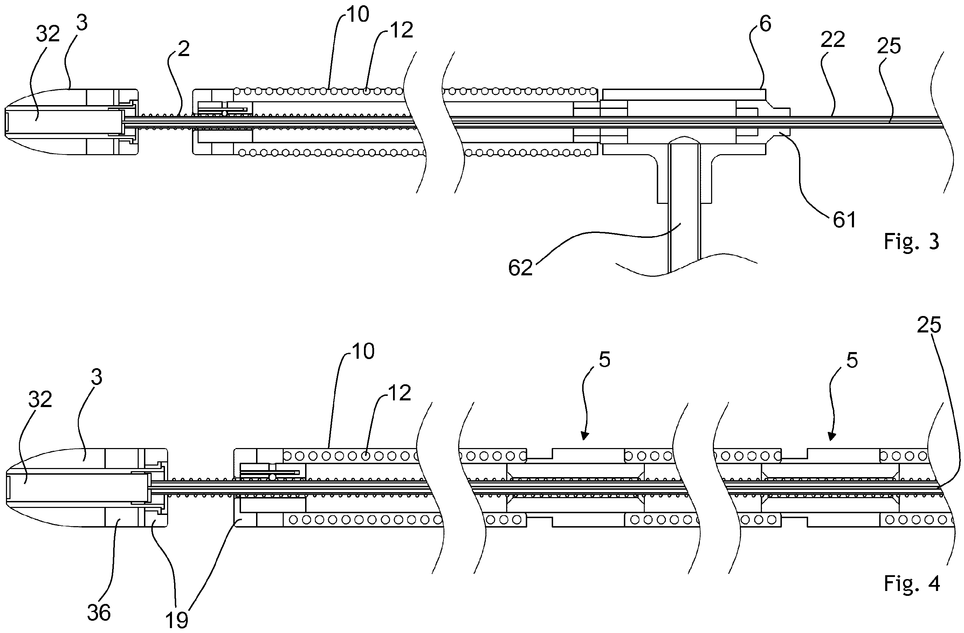

As can be seen in Fig. 1, the spiral reinforcement 12 may cause the outer surface of the tubular structure 10 to have a spiraling or helical structure. This helical structure can engage with the intestinal wall and aid in the forward movement of the endoscope during rotation of the tubular structure 10. While the endoscope according to the third embodiment shown in Fig. 3 also shows this helical structure due to the spiral reinforcement 12 impinging through the outer surface of the tubular structure 10, the embodiments shown in Fig. 2 and 4 show a smooth surface of the tubular structure 10. In these embodiments, the spiral reinforcement 12 is buried deeper in the flexible membrane material.

An endoscope according to a second embodiment is shown in Fig. 2. The endoscope comprises a sliding seal 61 at its proximal end 6, which seals the inner lumen 11 of the tubular structure 10, even when the elongated element 22, which proximally extends beyond the proximal end 6, moves along the longitudinal axis. The proximal end 6 is made of an end element with an inlet 62 for introducing gaseous or liquid substances into the inner lumen 11. The inlet 62 may be utilized to change the pressure inside the inner lumen 11, e.g. to pressurize or to depressurize it, in particular for activating any modules that are actuated by a change of pressure.

In this embodiment, the thread structure 2 is only provided on a distal section of the elongated element 22, and thus ends within the inner lumen 11 of the tubular structure 10. As the thread structure 2 only serves for providing the rotational engagement of between the tubular structure 10 and the elongated element 22, it is not necessary that it extends much beyond the engagement element 18 at the most possible or practical extension position of the endoscopic tip 3. This is also the case for the endoscope according to the third embodiment shown in Fig. 3. In addition, as explained earlier, the tubular structure 10 of the endoscope in Fig. 3 has a spiraling surface due to the spiral reinforcement 12, which is not completely buried in the flexible membrane material surrounding it. This is the only difference visible in the Fig. 2 and 3 between the endoscopes shown there.

Fig. 4 shows an endoscope according to a fourth embodiment. The endoscope comprises two modules 5, which are arranged at a distance along the tubular structure 10. These may be any kind of actuation and/or sensory modules. As the embodiment shown in Fig. 4 is only an exemplary one, there may of course me more than two such modules 5 arranged along the endoscope, which are not shown in Fig. 4 because Fig. 4 only shows three sections of the endoscope.

The endoscopic tip 3 of each of the endoscopes shown in Fig. 1-4 includes an endoscopic camera 32 for imaging the intestine while the endoscope advances into it. While this might also be the case in an alternative embodiment to that of Fig. 1, Figs. 2-4 explicitly show wires 25 connected to the camera 32 running through the central lumen 23 of the elongated element 22.

Each of the endoscopes shown in the Fig. 1-4 include a pair of distal electrodes 19, one of which is attached to the proximal end of the endoscopic tip 3 and the other to the distal end 14 of the tubular structure 10. By depressurizing the inside lumen 11 of the tubular structure 10, the volume inside the gap 34 between the endoscopic tip 3 and the distal end 14 may be depressurized as well due to the link formed by the distal opening 13, thus sucking in part of the intestinal wall. The endoscopic tip 3 can then be retracted towards the distal end 14 in order to sandwich a part of the intestinal wall between the pair of distal electrodes 19 for impedance measurement.

The endoscopic tips 3 shown in the embodiments in Fig. 2-4 each include a steering magnet 36, which can be utilized in order to steer the endoscopic tips 3 and thus the endoscope with the help of a magnetic field produced outside of the patient's body and penetrating the intestine.

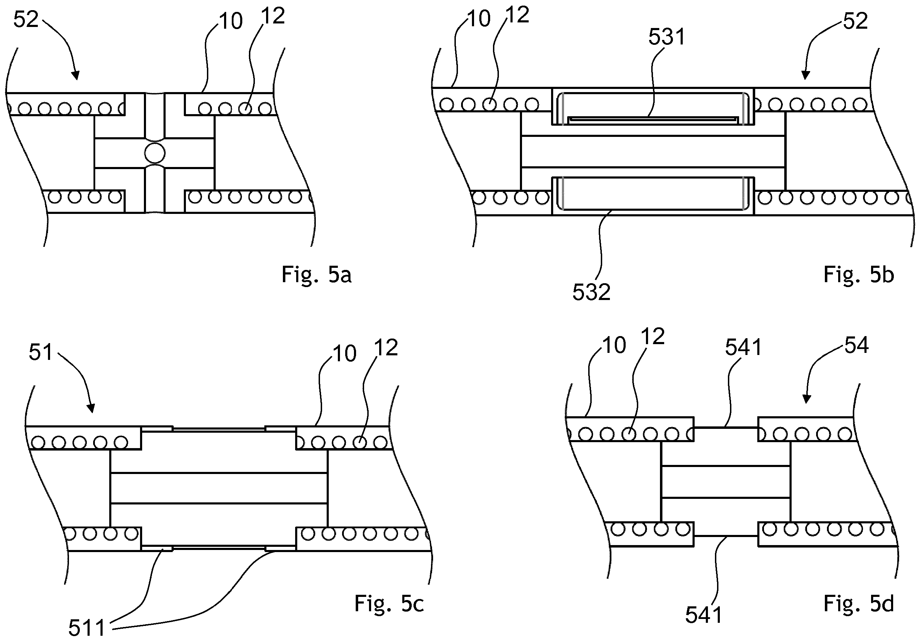

Figs. 5a-5d show schematic cross-section drawings of different actuation modules and sensory modules incorporated in a tubular structure 10 of an endoscope. The module shown in Fig. 5a, a fluid or drug distribution module 52, may be regarded as an actuation module. Upon activation, the fluid or drug distribution module 52 releases a substance into the intestine. The amount of the released substance may be fixed before the start of the endoscopic procedure, e.g. by filling the module 52 with a desired portion of the substance, all of which will be released simultaneously upon activation. Alternatively, the module 52 might be filled with a certain amount of the substance, a dosage of which is released upon activation, where the dosage may be controlled upon activation. Alternatively, the fluid or drug to be released may be pumped through the inner lumen 11 and flow out of the distribution module 52 or out of some or all the distribution modules 52, in case more than one such module 52 is provided.

Fig. 5b and 5c show sensory modules. In Fig. 5b, a camera module 53 is shown, which the endoscope may be equipped with, instead of or in addition to the endoscopic camera placed on the endoscopic tip. The camera module 53 is comprised of an image sensor 531 and a clear casing 532 protecting the image sensor 531. The camera module 53 may be equipped with more than one image sensor 531 for taking still or video images of different locations of the intestine and/or at different angles. Alternatively or in addition, the image sensor 531 or sensors (not shown) may be configured to be movable with respect to the camera module 53 or with respect to the tubular structure 10.

The sensory module shown in Fig. 5c is an impedance measurement module 51. For measuring in particular a transepithelial impedance in the intestine, the module 51 comprises two or more module electrodes 511, which are electrically insulated against each other. An impedance measuring circuit may be located in the impedance measurement module 51 or in a different position on the endoscope. Alternatively, wires may lead from the module electrodes 511 to the proximal end of the endoscope and be connected to an impedance measurement device located outside of the endoscope.

Fig. 5d shows a handling module 54, which is configured to hold or engage with a tool for handling the endoscope. The tool may preferably be configured for helping the physician to exert more torque on the tubular structure 10 and/or rotate it more quickly. As the movement into the small intestine is effected mainly by rotation of the tubular structure 10 in alternating rotation directions, and rotating the tubular structure 10 becomes increasingly cumbersome due to friction, the use of such a tool may greatly facilitate the work of the physician. The handling module 54 in the embodiment of Fig. 5d is equipped with two wrench flats 541 on opposite sides of the tubular structure 10, which allow a tool such as a wrench to be attached to it or engaged with it.

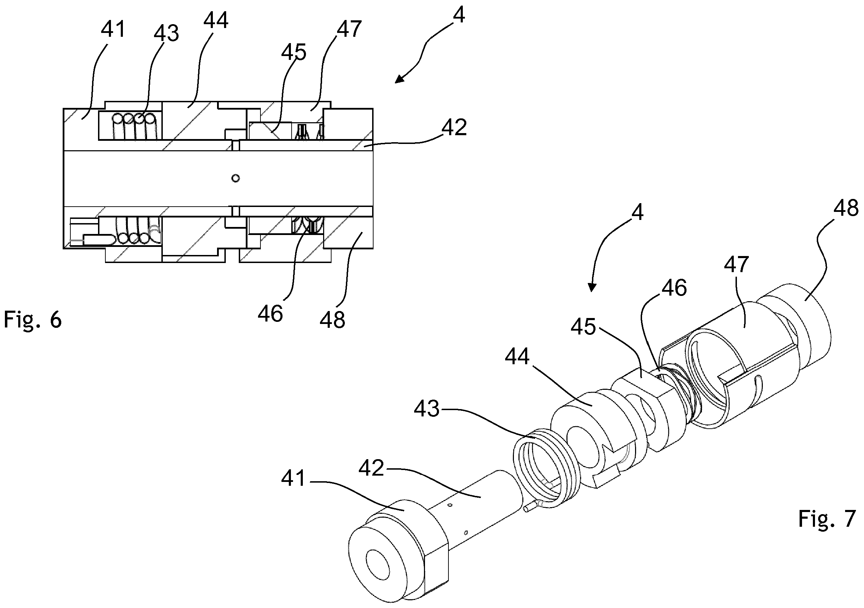

A preferred embodiment of a biopsy sampling module 4 is shown in a cross-sectional view in Fig. 6 and in an exploded view in Fig. 7. The biopsy sampling module 4 comprises the following parts arranged on a central shaft 42: a spring holder 41 holding a torsion spring 43, a sampling wheel 44 configured as a rotating blade 44, a piston 45, a piston spring 46, a casing 47, and an endcap 48. The central shaft 42 is configured to be fixed to the tubular structure 10. It has a lumen through which the elongated element 22 passes, and which is in fluid contact with the inner lumen 11 of the tubular structure 10. The torsion spring 43 is engaged with the sampling wheel 44, which in turn is free to rotate around the central shaft 42. The sampling wheel 44 has a cutout which may itself serve as a biopsy tool, or into which a disposable sample holder can be inserted and later removed.

The disposable sample holder (not shown in the figures) comprises a receptacle where the sample is stored, and a blade or brush with which the biopsy is obtained. The casing 47 surrounds the sampling wheel 44, guarding the disposable sample holder when not in use and protecting the sample holder from cross-contamination both before and after the sampling occurs. The casing 47 further has a cutout to allow the sampling blade or brush to contact the tissue when the module 4 is triggered. The piston 45 is fitted around the central shaft 42 just behind the sampling wheel 44 and is free to move linearly along the shaft axis. The piston spring 46 pushes the piston 45 up against the sampling wheel 44, locking the sampling wheel 44 in place. The piston 45 has a pin (not shown) to prevent the rotation of the sampling wheel 44.

When the lumen of the sampling module 4 is pressurized, holes in the central shaft 42 allow the air to push the piston 45 back against the piston spring 46, releasing the sampling wheel 44 and causing the biopsy to be taken. At the end of its stroke, the sample holder has rotated by approximately 180 degrees and is now protected from contamination by the other side of the casing 47. When the pressure is released, the piston 45 returns to its original position, fixing the sampling wheel 44 in its final position. Pressurizing and depressurizing the Sampling module 4 in order to displace the piston 45 is performed by pressurizing and depressurizing the inside lumen 11 of the tubular structure 10 by way of the inlet 62. The endcap 48 is fixed to the central shaft 42 as well as to the tubular structure 10.

The endoscope is equipped with a number of sampling modules 4 periodically disposed along its length for the purpose of obtaining biopsies or scraping of the epithelial tissue. These sampling modules 4 are pre-loaded and can be triggered simultaneously, ensuring that biopsies are taken at regular distance intervals along the intestine. The biopsy sampling modules 4 may for example be arranged at regular intervals of between 20 cm and 25 cm. In this case, there may be between 24 and 30 such modules provided along a 6 m length of the tubular structure 10.

### Reference Numerals:

- 10: tubular structure
- 11: inner lumen of tubular structure
- 12: spiral reinforcement
- 13: distal opening
- 14: distal end
- 16: bearing ball cover [sleeve]
- 18: bearing ball
- 19: distal electrodes

- 2: thread structure
- 22: elongated element
- 23: central lumen of elongated element
- 25: wires

- 3: endoscopic tip
- 32: camera
- 34: gap
- 36: steering magnet

- 4: biopsy sampling module
- 41: spring holder
- 42: shaft
- 43: torsion spring
- 44: rotating blade
- 45: piston
- 46: piston spring
- 47: casing
- 48: endcap

- 5: modules
- 51: impedance measurement module
- 511: module electrodes
- 52: fluid or drug delivery module
- 53: camera module
- 531: image sensor
- 532: clear case
- 54: handling module

- 6: proximal end
- 61: sliding seal
- 62: inlet

## Claims

1. Endoscope for inspection of the gastrointestinal tract of a mammal, in particular of the small intestine of a human, comprising an elongated, flexible tubular structure (10) surrounding an inner lumen (11) and ending in a distal end (14) having a distal opening (13) and a flexible, elongated element (22) placed inside the tubular structure (10) and running through the inner lumen (11) and the distal opening (13) such that a distal portion (23) of the elongated element (22) protrudes from the distal end (14) of the tubular structure (10) and is connected to an endoscopic tip (3), wherein the elongated element (22) is engaged with the tubular structure (10) in such way that a rotation of the tubular structure (10) around the elongated element (22) in a first rotation direction (R1) will increase a gap (34) between the endoscopic tip (3) and the distal end (14), while a rotation of the tubular structure (10) around the elongated element (22) in a second rotation direction (R2), opposite to the first rotation direction (R1), will decrease the gap (34) between the endoscopic tip (3) and the distal end (14), wherein said endoscopic tip (3) is in a retracted position when said elongated element (22) is retracted as far as possible into the tubular structure (10), such that the endoscopic tip (3) is abutting the distal end (14) of the tubular structure (10), wherein said endoscopic tip (3) is configured to seal the inner lumen (11) at the distal opening (13) of said tubular structure (10) and allowing said inner lumen (11) to be pressurized or depressurized by way of covering said distal opening (13) when said endoscopic tip (3) is in the retracted position, **characterized in that** said distal end (14) of an inner lumen (11) of said tubular structure (10) is not sealed or not completely sealed from the outside as long as the endoscopic tip (3) is not completely retracted.

2. Endoscope according to claim 1, wherein an electrode (19) is disposed on a proximal side of the endoscopic tip (3) and a corresponding counter-electrode (19) is disposed on a distal end (14) of said tubular structure (10), such that the two electrodes (19) are separated by the gap (34) between the endoscopic tip (3) and the distal end (14).

3. Endoscope according to one of the previous claims, wherein said engagement between the elongated element (22) and the tubular structure (10) is by way of an engaging element (18) engaging with a thread structure (2).

4. Endoscope according to claim 3, wherein said thread structure (2) is a thread formed on an outside surface of said elongated element (22).

5. Endoscope according to claim 3 or 4, wherein said engaging element is a protruding element, which reaches into the threads of the thread structure (2) in order to translate a rotation of the tubular structure (10) into a translational movement of the elongated element (22) with respect to the engaging element.

6. Endoscope according to claim 5, wherein said engaging element is a pin, a peg, or a bearing ball (18) placed inside the distal end of the tubular structure.

7. Endoscope according to one of the previous claims, wherein said engagement between the elongated element (22) and the tubular structure (10) is effected at two or more positions along the length of the elongated element (22) and/or the tubular structure (10).

8. Endoscope according to one of the previous claims, wherein said elongated element (22) and said tubular structure (10) are arranged coaxially.

9. Endoscope according to one of the previous claims, wherein said tubular structure (10) comprises a spiraling surface structure.

10. Endoscope according to one of the previous claims, wherein said endoscopic tip (3) has substantially the same diameter as said distal end (14) of said tubular structure (10).

11. Endoscope according to one of the previous claims, wherein said endoscopic tip (3) comprises a camera (32).

12. Endoscope according to one of the previous claims, comprising one or multiple actuation and/or sensory module(s).

13. Endoscope according to claim 12, wherein said actuation and/or sensory module(s) is/are from the group of a biopsy sampling module (4), an impedance measurement module (51), a fluid or drug delivery module (52), a camera module (53), a handling module (54) for handling or holding the endoscope, or any combination thereof.

14. Endoscope according to claim 12 or 13, comprising multiple actuation and/or sensory modules arranged at regular intervals along the length of said tubular structure (10).

15. Endoscope according to one of the claims 12 or 14, wherein one, multiple or all of said actuation modules are pneumatically or hydraulically actuated and configured to be activated by a change of pressure of an inner lumen of said tubular structure (10).

## Patentansprüche

1. Endoskop zur Untersuchung des Magen-Darm-Trakts eines Säugetiers, insbesondere des Dünndarms eines Menschen, enthaltend eine langgestreckte, flexible rohrförmige Struktur (10), die ein inneres Lumen (11) umgibt und in einem distalen Ende (14) mit einer distalen Öffnung (13) endet, und ein flexibles, langgestrecktes Element (22), das in der rohrförmigen Struktur (10) angeordnet ist und durch das innere Lumen (11) und die distale Öffnung (13) geht, so dass ein distaler Abschnitt (23) des langgestreckten Elements (22) von einem distalen Ende (14) der rohrförmigen Struktur (10) vorspringt und mit einer endoskopischen Spitze (3) verbunden ist, wobei das langgestreckte Element (22) in die rohrförmige Struktur (10) derart eingreift, dass eine Drehung der rohrförmigen Struktur (10) um das langgestreckte Element (22) in einer ersten Drehrichtung (R1) einen Spalt (34) zwischen der endoskopischen Spitze (3) und dem distalen Ende (14) vergrößern wird, während eine Drehung der rohrförmigen Struktur (10) um das langgestreckte Element (22) in einer zweiten Drehrichtung (R2), entgegengesetzt zur ersten Drehrichtung (R1), den Spalt (34) zwischen der endoskopischen Spitze (3) und dem distalen Ende (14) verringern wird, wobei sich die endoskopische Spitze (3) in einer eingefahrenen Position befindet, wenn das langgestreckte Element (22) so weit wie möglich in die rohrförmige Struktur (10) eingefahren ist, so dass die endoskopische Spitze (3) an das distale Ende (14) der rohrförmigen Struktur (10) anstößt, wobei die endoskopische Spitze (3) konfiguriert ist, das innere Lumen (11) an der distalen Öffnung (13) der rohrförmigen Struktur (10) abzudichten, und ermöglicht, dass das innere Lumen (11) mittels Abdeckens der distalen Öffnung (13) unter Druck gesetzt oder drucklos gemacht wird, wenn die endoskopische Spitze (3) sich in der eingefahrenen Position befindet,
**dadurch gekennzeichnet, dass** das distale Ende (14) eines inneren Lumens (11) der rohrförmigen Struktur (10) von außen nicht oder nicht vollständig abgedichtet ist, solange die endoskopische Spitze (3) nicht vollständig eingefahren ist.

2. Endoskop nach Anspruch 1, wobei eine Elektrode (19) auf einer proximalen Seite der endoskopischen Spitze (3) angeordnet ist und eine entsprechende Gegenelektrode (19) an einem distalen Ende (14) der rohrförmigen Struktur (10) angeordnet ist, so dass die zwei Elektroden (19) durch den Spalt (34) zwischen der endoskopischen Spitze (3) und dem distalen Ende (14) getrennt sind.

3. Endoskop nach einem der vorhergehenden Ansprüche, wobei der Eingriff zwischen dem langgestreckten Element (22) und der rohrförmigen Struktur (10) über ein Eingriffselement (18) erfolgt, das in eine Gewindestruktur (2) eingreift.

4. Endoskop nach Anspruch 3, wobei die Gewindestruktur (2) ein Gewinde ist, das auf einer Außenfläche des langgestreckten Elements (22) gebildet ist.

5. Endoskop nach Anspruch 3 oder 4, wobei das Eingriffselement ein vorspringendes Element ist, das in die Gewinde der Gewindestruktur (2) greift, um eine Drehung der rohrförmigen Struktur (10) in eine Translationsbewegung des langgestreckten Elements (22) in Bezug auf das Eingriffselement zu übertragen.

6. Endoskop nach Anspruch 5, wobei das Eingriffselement ein Stift, ein Zapfen oder eine Lagerkugel (18) ist, die in dem distalen Ende der rohrförmigen Struktur angeordnet ist.

7. Endoskop nach einem der vorhergehenden Ansprüche, wobei der Eingriff zwischen dem langgestreckten Element (22) und der rohrförmigen Struktur (10) an zwei oder mehr Positionen entlang der Länge des langgestreckten Elements (22) und/oder der rohrförmigen Struktur (10) bewirkt wird.

8. Endoskop nach einem der vorhergehenden Ansprüche, wobei das langgestreckte Element (22) und die rohrförmige Struktur (10) koaxial angeordnet sind.

9. Endoskop nach einem der vorhergehenden Ansprüche, wobei die rohrförmige Struktur (10) eine spiralförmige Oberflächenstruktur aufweist.

10. Endoskop nach einem der vorhergehenden Ansprüche, wobei die endoskopische Spitze (3) im Wesentlichen den gleichen Durchmesser aufweist wie das distale Ende (14) der rohrförmigen Struktur (10).

11. Endoskop nach einem der vorherigen Ansprüche, wobei die endoskopische Spitze (3) eine Kamera (32) enthält.

12. Endoskop nach einem der vorhergehenden Ansprüche, enthaltend ein oder mehrere Betätigungs- und/oder Sensormodul(e).

13. Endoskop nach Anspruch 12, wobei das/die Betätigungs- und/oder Sensormodul(e) aus der Gruppe eines Biopsie-Probenahmemoduls (4), eines Impedanzmessmoduls (51), eines Fluid- oder Wirkstoffabgabemoduls (52), eines Kameramoduls (53), eines Handhabungsmoduls (54) für die Handhabung oder das Halten des Endoskops oder eine Kombination davon ist.

14. Endoskop nach Anspruch 12 oder 13, enthaltend mehrere Betätigungs- und/oder Sensormodule, die in regelmäßigen Abständen entlang der Länge der rohrförmigen Struktur (10) angeordnet sind.

15. Endoskop nach einem der Ansprüche 12 oder 14, wobei ein, mehrere oder alle Betätigungsmodule pneumatisch oder hydraulisch betätigt und konfiguriert sind, durch eine Druckänderung eines inneren Lumens der rohrförmigen Struktur (10) aktiviert zu werden.

## Revendications

1. Endoscope pour l'inspection du tractus gastro-intestinal d'un mammifère, en particulier de l'intestin grêle d'un humain, comprenant une structure tubulaire allongée et flexible (10) entourant une lumière interne (11) et finissant dans une extrémité distale (1) ayant une ouverture distale (13) et un élément flexible et allongé (22) placé à l'intérieur de la structure tubulaire (10) et passant par la lumière interne (11) et l'ouverture distale (13) de telle sorte qu'une partie distale (23) de l'élément allongé (22) saillie à partir de l'extrémité distale (14) de la structure tubulaire (10) et est relié à une pointe endoscopique (3), dans lequel l'élément allongé (22) est engagé avec la structure tubulaire (10) de telle sorte qu'une rotation de la structure tubulaire (10) autour de l'élément allongé (22) dans un premier sens de rotation (R1) augmentera un écart (34) entre l'extrémité endoscopique (3) et l'extrémité distale (14), tandis qu'une rotation de la structure tubulaire (10) autour de l'élément allongé (22) dans un deuxième sens de rotation (R2), opposé au premier sens de rotation (R1), réduira l'écart (34) entre l'extrémité endoscopique (3) et l'extrémité distale (14), dans lequel ladite pointe endoscopique (3) est en position rétractée lorsque ledit élément allongé (22) est rétracté autant que possible dans la structure tubulaire (10), de telle sorte que la pointe endoscopique (3) bute contre l'extrémité distale (14) de la structure tubulaire (10), dans lequel ladite pointe endoscopique (3) est configurée pour sceller une lumière interne (11) à l'ouverture distale (13) de ladite structure tubulaire (10) et permet à ladite lumière interne (11) d'être pressurisée ou dépressurisée en recouvrant ladite ouverture distale (13) lorsque ladite pointe endoscopique (3) est en position rétractée,
**caractérisé en ce que** ladite extrémité distale (14) d'une lumière interne (11) de ladite structure tubulaire (10) n'est pas scellée ou n'est pas complètement scellée de l'extérieur tant que l'extrémité endoscopique (3) n'est pas complètement rétractée.

2. Endoscope selon la revendication 1, dans lequel une électrode (19) est disposée sur une face proximale de l'extrémité endoscopique (3) et une contre-électrode correspondante (19) est disposée sur une extrémité distale (14) de ladite structure tubulaire (10), de telle sorte que les deux électrodes (19) sont séparées par l'espace (34) entre l'extrémité endoscopique (3) et l'extrémité distale (14).

3. Endoscope selon l'une des revendications précédentes, dans lequel ledit engagement entre l'élément allongé (22) et la structure tubulaire (10) s'effectue par un élément engageant (18) s'engageant avec une structure de filet (2).

4. Endoscope selon la revendication 3, dans lequel ladite structure de filet (2) est un filet formé sur une surface extérieure dudit élément allongé (22).

5. Endoscope selon la revendication 3 ou 4, dans lequel ledit élément engageant est un élément saillant, qui atteint les filets de la structure de filet (2) afin de convertir une rotation de la structure tubulaire (10) en un mouvement de translation de l'élément allongé (22) par rapport à l'élément engageant.

6. Endoscope selon la revendication 5, dans lequel ledit élément engageant est une goupille, une cheville, ou une bille de palier (18) placée à l'intérieur de l'extrémité distale de la structure tubulaire.

7. Endoscope selon l'une des revendications précédentes, dans lequel ledit engagement entre l'élément allongé (22) et la structure tubulaire (10) est effectué à deux ou plusieurs positions le long de l'élément allongé (22) et/ou de la structure tubulaire (10).

8. Endoscope selon l'une des revendications précédentes, dans lequel ledit élément allongé (22) et ladite structure tubulaire (10) sont disposés coaxialement.

9. Endoscope selon l'une des revendications précédentes, dans lequel ladite structure tubulaire (10) comprend une structure de surface en spirale.

10. Endoscope selon l'une des revendications précédentes, dans lequel ladite pointe endoscopique (3) a sensiblement le même diamètre que ladite extrémité distale (14) de ladite structure tubulaire (10).

11. Endoscope selon l'une des revendications précédentes, dans lequel ladite pointe endoscopique (3) comprend une caméra (32).

12. Endoscope selon l'une des revendications précédentes, comprenant un ou plusieurs modules d'actionnement et/ou sensoriels.

13. Endoscope selon la revendication 12, dans lequel le(s)dit(s) module(s) d'actionnement et/ou sensoriels est/sont du groupe d'un module de prélèvement de biopsie (4), d'un module de mesure d'impédance (51), d'un module d'administration de fluide ou de médicament (52), d'un module de caméra (53), d'un module de manipulation (54) pour manipuler ou tenir l'endoscope, ou toute combinaison de ceux-ci.

14. Endoscope selon la revendication 12 ou 13, comprenant de multiples modules d'actionnement et/ou sensoriels disposés à intervalles réguliers sur la longueur de ladite structure tubulaire (10).

15. Endoscope selon l'une des revendications 12 ou 14, dans lequel un, plusieurs ou tous lesdits modules d'actionnement sont actionnés pneumatiquement ou hydrauliquement et configurés pour être activés par un changement de pression d'une lumière interne de ladite structure tubulaire (10).
